# EUROPEAN PATENT SPECIFICATION

(11) **EP 3 125 880 B1**
(45) Date of publication and mention of the grant of the patent: **20.11.2019**
(21) Application number: 15713330.7
(22) Date of filing: 30.03.2015
(51) Int. Cl.: A61K 31/192, A61K 31/327, A61P 17/02

(54) **COMBINATION OF ADAPALENE AND BENZOYL PEROXIDE FOR PREVENTING ACNE SCARS**
KOMBINATION AUS ADAPALEN UND BENZOYLPEROXID ZUR VORBEUGUNG VON ATROPHISCHEN AKNENARBEN
COMBINAISON D'ADAPALÈNE ET DE PEROXYDE DE BENZOYLE POUR PRÉVENTION DES CICATRICES ATROPHIQUES DUES À L`ACNÉ

(30) Priority: 01.04.2014 US 201461973350 P
(43) Date of publication of application: 08.02.2017
(73) Proprietor: Galderma Research & Development, 06410 Biot (FR)
(72) Inventor: BOURDES, Valérie, F-06600 Antibes (FR); MARTEL, Philippe, F-06410 Biot (FR); RIVIER, Michel, F-06100 Nice (FR); REYNIER, Philippe, F-06560 Valbonne (FR); PETIT, Laurent, F-06530 Peymeinade (FR)
(74) Representative: Casalonga
(86) International application number: PCT/EP2015/056906
(87) International publication number: WO 2015/150332

(56) References cited:
- WO-A1-2008/006888
- WO-A1-2008/092911
- WO-A1-2011/098391
- WO-A1-2011/135090
- WO-A1-2015/091828
- US-A1- 2009 191 245

## Description

Acne vulgaris is a chronic, inflammatory skin disease of the pilosebaceous unit, affecting approximately 80% of young adults and adolescents (Leyden JJ.et al. New understandings of the pathogenesis of acne. J Am Acad Dermatol. 1995;32:S15-S25). Even if the treatments of acne are well known and efficient, in many cases scarring is the most unfortunate clinical outcome of acne (Layton AM,et al. A clinical evaluation of acne scarring and its incidence. Clinical and Experimental Dermatology 1994; 19:303-308). Acne scars are not uniform and there are several subtypes. Some are more hypertrophic and keloidal in appearance, while others could be more atrophic (Goodman GJ, et al. Postacne scarring - a quantitative global scarring grading system. Journal of Cosmetic Dermatology 2006;5:48-52). The severity of the acne scars is correlated with the acne grade and also with the delay between the start of the disease and the start of an adapted treatment. Although not universal, acne scars generally occur with more inflammatory acne lesions that were not properly treated (Layton AM,et al. Scarred for life? Dermatology 1997; 195(suppl 1):15-21). Once established, acne scars are believed not to be treatable medically but invasive procedures could offer some improvement (Jemec GBE, Jemec B. Acne: Treatment of Scars. Clinics in Dermatology 2004;22:434-438).

Loss of dermal matrix has to be a contributing factor in atrophic acne scars (Kang S, et al. Inflammation and extracellular matrix degradation mediated by activated transcription factors NFkB and AP-1 in inflammatory acne lesions in vivo. Am J Pathol 2005;166:1691-1699) . This loss of dermal matrix consists in degradation of collagen produced by intracellular pathway activated by inflammation during the acne lesion formation. This leads to transcription factor activation coding for matrix-degrading metalloproteinases (MMPs). MMPs degrade extracellular matrix molecules during physiological and pathological tissue remodeling. Three MMPs (MMP-1 [collagenase], MMP-3 [stromelysin] that can initiate collagen degradation and MMP-9 [gelatinase] that can further degrade collagen fragments) have been shown to be increased in inflammatory acne lesion in vivo (Papakonstantinou E, et al. Matrix Metallonproteinases of epithelial origin in facial sebum of patients with acne and their regulations by isotretinoin. J Invest Dermatol. 2005;125:673-684. and also Trivedi NR et al. Gene Array Expression Profiling in Acne Lesions. J of Invest Dermatol. 2006;126(5):1071-1079.

The same enzymes are involved in the remodeling of the dermal matrix in the photoaging process (Kang S, Fisher GJ, Voorhees JJ. Photoaging and topical tretinoin: therapy, pathogenesis, and prevention. Arch Dermatol. 1997;133:1280-1284.. It has been demonstrated that UV radiation activates transcription factor that lead to collagen degradation (Fisher GJ, Datta S, Wang ZQ, et al. c-Jun-dependent inhibition of cutaneous procollagen transcription following ultraviolet irradiation is reversed by all-trans retinoic acid. J Clin Invest 2000;106:663-670.Involved proteins are the same as for inflammatory acne lesions (collagenase, stromelysin respectively MMP-1, 3 and gelatinase, MMP-9) (Kang S, Voorhees JJ. Photoaging therapy with topical tretinoin: an evidence-based analysis. J Am Acad Dermatol. 1998;39:S55-61).

Over the past decades, significant evidence has been accumulated that topical retinoids can stimulate dermal fibroblasts to produce more procollagen in photoaged skin. Type I and type III procollagen are reduced in photodamage skin but it has been demonstrated that isotretinoin, either oral or topical, protects against UV-induced loss of procollagen, leading to MMP-1, 3 and MMP-9 decrease in relation to the duration of the treatment (Kang S, Voorhees JJ. Photoaging therapy with topical tretinoin: an evidence-based analysis. J Am Acad Dermatol. 1998;39:S55-61).

This increase in collagen production is clinically appreciated as effacement of wrinkles. After 24 weeks of treatment with topical retinoid, an increase of the epidermal thickness has been observed (Griffiths CEM, et al. Two concentrations of topical tretinoin (retinoic acid) cause similar improvement of photoaging but different degrees of irritation: a double-blind, vehicle-controlled comparison of tretinoin 0.1% and 0.025% creams. Arch Dermatol. 1995;131:1037-1044).

Adapalene Gel 0.1% (Differin®) is approved in USA for the treatment of acne and has been found to be as effective as other retinoids. It was shown to have a favorable tolerability profile in comparison with other retinoids. Adapalene Gel 0.1% and 0.3% formulations have been demonstrated to treat actinic keratoses, and solar lentigines and improve some of the effects of photodamage such as fine and coarse wrinkles, mottled hyperpigmentation, and sallow complexion (Kang S, Goldfarb MT, Weiss JS, et al. Assessment of Adapalene gel for the treatment of actinic keratoses and lentigines: A randomized trial. J Am Acad Dermatol. 2003; 49:1; 83-90.

As atrophic acne scars may cause serious physical and emotional scarring and can significantly impact the quality of life (Koo JY, Smith LL. Psychologic aspects of acne. Pediatr Dermatol. 1991, 8(3):185-188) the aim of the present invention is to provide a method for treating scars and particularly acne scars. In addition the present invention provides a method for preventing papules to evolve in scars, and thus preventing acne scars appearance.

Scars are marks created during the healing of damage to the skin or tissues .A scar is permanent and cannot be completely removed. However, treatment can alter a scar's appearance. These procedures range from the application of over-the-counter ointment to surgery. Scar treatment should start after an injury because wound care affects scarring. The wound should be cleaned and covered. Picking at the scab breaks the collagen and allows germs to enter the wound. Time also helps with healing. Scars become smaller, and the color fades.

However, additional treatment is required for some scars. While some procedures are more effective for keloids and hypertrophic scars, the procedure for acne scars is based on the type of scarring.

Therefore, there is a need of an effective treatment treating acne scars and/or preventing their setting up or appearance. Preferably, there is a need of an effective treatment treating atrophic acne scars and/or preventing their setting up or appearance

Hence, the first object of the present invention is preventing acne atrophic scars where an effective amount of a fixed combination of adapalene or its salts and benzoyl peroxide (BPO) is administrated to the patient in need thereof, over a period treatment of 24 weeks. Preferably adapalene is used in a quantity of 0.1% of weight relative to the total weight of the composition, but this concentration can be in the range of about 0.1% to about 0.3% by weight.

The benzoyl peroxide (BPO) is administered preferentially in a quantity of 2.5% of weight relative to the total weight of the composition, but this concentration can be in the range of about 2.5% to about 5% by weight.

All the pharmaceutical compositions administered in accordance with the invention can comprise from about 0.01% to about 2%, preferably from about 0.05% to about 0.5% and preferentially from about 0.1% to about 0.3% of adapalene, and from about 0.1% to less than about 5% and preferably from about 0.5% to less than about 5% of BPO, more preferably from about 2% to less than about 5% of BPO and preferentially about 2.5% of BPO, all amounts being expressed by weight with regard to the total weight of the composition.

The effective amount of adapalene and of BPO is in a composition and preferably in the form of an aqueous gel. The scars prevented are acne atrophic scars. More specifically, acne scar is selected from ice pick, boxcar, rolling, bridges and tunnels and gross atrophy.

According to one specific embodiment, the effective amount of adapalene and BPO is topically administrated and more specifically are applied onto scared skin. In a preferred embodiment it is topically administrated, preferentially onto the skin at 0.1% of weight relative to the total weight of the composition for adapalene and at 2.5% of weight relative to the total weight of the composition for BPO.

In another embodiment, the invention concern the use of adapalene at a range of 0.1% to 0.3% and BPO at a range of 2.5% to 5%, for the preparation of a medicament which is intended for the prevention of acne atrophic scars. Accordingly said use is characterized in that the medicament is in the form of a composition suitable for topical application. Preferentially the medicament is in the form of a gel, emulsion, lotion.

In another embodiment, the present invention concerns a composition containing a fixed combination of adapalene or a salt thereof and benzoyl peroxide for its use for preventing acne atrophic scars over a period treatment of 24 weeks.

Even with the most careful and conscientious treatment, acne scars may still occur. But not all scars are created equal. Generally, acne scars fall under two categories: those caused by a loss of tissue (atrophic), and those caused by an excess of tissue (hypertrophic). Within these categories, acne scars fall into one of four types: ice pick, boxcar, rolling and keloid scars.

Prior art provides definition of acne scars (Alam M, Dover JS. "Treatment of Acne Scarring." Skin Therapy Letter. Dec 2006-Jan 2007; 11(10); Goodman GJ, Baron JA. "The management of post-acne scarring." Dermatologic Surgery. Oct 2007; 33(10):1175-1188.; Jacob CI, Dover JS, Kaminer MS. "Acne scarring: a classification system and review of treatment options." Journal of the American Academy of Dermatology. 2001; 45(1): 109-117.):
- Ice pick scars are deep, very narrow scars that extend into the dermis. The skin looks as if it has been pierced by an ice pick or sharp instrument. Ice pick scars seem to make a small, deep "hole" in the skin. Some may look like a large, open pore. Ice pick scars develop after an infection from a cyst or other deep inflamed blemish works its way to the surface. Skin tissue is destroyed, leaving a long column-like scar. Ice pick scars can commonly be treated with punch excision or punch grafting.
- Boxar scars are depressed acne scars, round or oval in shape with steeply angled sides. They are similar in appearance to chickenpox scars. Boxcar scars occur when an inflamed acne lesion destroys tissue, leaving a sunken area on the skin. Boxcar scars may be mild and superficial, or deeper and more severe.

When an inflammatory breakout destroys collagen, tissue is lost. The skin over this area is left without support, and a depressed area is created. Boxcar scars may be superficial to severe, depending on the amount of tissue lost. The common treatments for boxcar scars include punch excision or elevation, dermal fillers, and laser resurfacing.
- Rolling scars arise when fibrous bands of tissue develop between the skin and the subcutaneous tissue below. These bands pull the epidermis, binding it to deeper structures of the skin. It is this pulling of the epidermis from within that creates the rolling appearance of the skin. This type of scarring causes rolling or "wave-like" undulations across otherwise normal appearing skin. Rolling scars are best treated with subcision.
- Unlike ice pick or boxcar scars, keloid scars are hypertrophic scars are not caused by a loss of tissue. Rather, they develop because of an overproduction of collagen. The common treatments are selected from: Steroid (cortisone) creams, tapes, or injections to help shrink and flatten the scar. Interferon injections are also used to soften scar tissue. A hypertrophic scar looks like a raised, firm mass of tissue. These types of scars often grow larger than the original wound. Hypertrophic scars caused by acne are most often found on the torso, especially in men.

A scar is a manifestation of the skin's healing process. After skin or tissue is wounded, the body releases collagen to mend the damage. Scarring is the natural process of repairing an open wound, injury, surgical incision, or other conditions like acne. Causes of scars include cuts, sores, surgery, and burns. Severe acne, varicella and chicken pox may also scar the skin. Therefore, the present invention encompasses not only scars induced by acne, in particular severe acne, but also other types of scars like those mentioned above.

In a preferred embodiment, the effective amount of the fixed combination is topically administrated and particularly is applied on scared skin.

In the context of the invention, it is meant by « effective amount» the quantity or the concentration of adapalene and BPO in the composition to provide the desired effect. Hence, any formulation of adapalene and BPO is usable in the context of the invention such as a gel, an emulsion or a solution.

Another embodiment regards the use of the fixed combination of adapalene or its salts and BPO for the preparation of a medicament which is intended for the prevention of acne atrophic scars. Advantageously, the medicament according to the present invention is intended for topical application.

The medicament according to the present invention also comprises a physiologically acceptable medium, that is to say a medium which is compatible with the skin, including the scalp, mucous membranes, hair, body hairs and/or eyes, and can constitute a dermatological composition.

The present invention uses the compound 6-[3-(1-Adamantyl)-4-methoxyphenyl]-2-naphthoic acid (referred to hereinbelow as adapalene) which is a naphthoic acid derivative with retinoid and anti-inflammatory properties as well as its salts.

The term "adapalene salts" means the salts formed with a pharmaceutically acceptable base, especially mineral bases such as sodium hydroxide, potassium hydroxide and ammonia or organic bases such as lysine, arginine or N-methylglucamine. The term "adapalene salts" also means the salts formed with fatty amines such as dioctylamine and stearylamine.

The treatment period is 24 weeks.

As an example of a fixed combination of adapalene and benzoyl peroxide (BPO) that is particularly suitable for use in the present invention, mention can be made of the product sold under the brand name Epiduo® at a weight concentration of 0.1% of adapalene and 2.5% of BPO, in the form of an aqueous gel. This composition is intended for treating acne. Patent application WO200 03/055472 describes gel compositions with adapalene and BPO and a pH-independent gelling agent for treating acne.

The term "fixed combination" should be understood as meaning a combination whose active principles are combined at fixed doses in the same vehicle (single formula) that delivers them together to the point of application.

The medicament for use in the present invention is a composition which can be in any pharmaceutical form normally used for topical application, such as aqueous dispersions, aqueous or oily suspensions, aqueous gels, anhydrous or lipophilic emulsions (lotions, creams or ointments) of liquid, semisolid or solid, obtained by dispersing a fatty phase in an aqueous phase (O / W) or conversely (W / H) in the presence or absence of emulsifier, or micro emulsions , micro capsules, micro particles or vesicular dispersions of ionic and / or nonionic.

Thus, in one preferred embodiment, the medicament according to the present invention can be provided in any pharmaceutical dosage form normally used in the field of dermatology.

Preferably, the medicament will be provided in the emulsion (lotions, creams, cream without emulsifier), suspensions or gel forms.

Preferably, the pharmaceutical composition in the form of a fixed combination is a gel; in this case, the two active principles are dispersed and intimately mixed, during production, in the same vehicle, which delivers them together during the application of the gel.

Useful pharmaceutical compositions, comprising adapalene and BPO, are moreover described in WO 03/055 472. Examples of such compositions comprise, besides the active principles adapalene and BPO:
- from about 5% to about 25% by weight of water;
- from about 0% to about 10%, preferably from about 0% to about 2% and preferably less than about 0.5% by weight of liquid wetting surfactant;
- from about 0% to about 10% by weight of pro-penetrating agent; and
- an aqueous phase comprising a pH-independent gelling agent.

Particularly, the composition comprising adapalene or its salts and BPO according to the present invention, comprises the following ingredients by weight relative to the total weight of the composition:
- adapalene: from 0,1% to 0,3%
- BPO: from 2.5% to 5%,
- at least one gelling agent: from 0.001% to 15% and more preferentially from 0.15% to 5%.
- at least one chelating agent: from 0,01% to 1,5%
- at least one preservative agent: from 0,01% to 3%
- at least one wetting agent: from 0.1% to 10%
- at least one pH adjuster.

Among the chelating agents, it can be mentionned as non limiting examples ethylene diamine tetraacetic acid (EDTA), diethylene triamine pentaacetic acid (DTPA), ethylene diaminetetraacetic acid di-(O-hydroxyphenyl acetic acid) (EDDHA) acid, hydroxy-2-ethylene diamine triacetic acid (HEDTA), ethyldiamine acid-di (O-methyl-p-hydroxy phenyl) acetic acid (EDDHMA) acid and ethylene diamine-di (5-carboxy-2 -hydroxyphenyl) acetic acid (EDDCHA).

The preferred chelating agent is ethylenediaminetetraacetic acid (EDTA), such as the product sold in particular under the name Titriplex III ®

Examples of gelling or suspending agents that may come in the compositions of the invention include , the so-called carbomers sold under the generic name of Carbopol® ; the carbomers sensitive to electrolytes, sold under the name ETD Carbopol 'Ultrez 10® by BF Goodrich; the polysaccharides such as xanthan gum as Keltrol® sold by Kelco, guar gum, chitosans, cellulose and its derivatives such as hydroxyethylcellulose, in particular the product sold under the name Natrosol HHX 250 by Aqualon Company; the polycrylamides like those sold under the tradenames Simulgel 600 (Acrylamide / Sodium Acryloyldimethyl Taurate Copolymer and Isohexadecane and Polysorbate 80) or Sepigel 305 (Polyacrylamide and C13-14 Isoparaffin and Laureth-7 °).

Preferred gelling agents are the carbomers sold in particular under the names Carbopol 974P NF and Carbopol 980 NF.

Among the preservatives or preservative agents mention is made as non-limiting examples of benzoic acid and its derivatives with benzyl alcohol, benzalkonium chloride, sodium benzoate, bronopol, chlorhexidine, chlorocresol and its derivatives, ethyl alcohol, phenethyl alcohol, phenoxyethanol, potassium sorbate, diazolidinylurea parabens such as propyl paraben or methyl paraben, alone or in mixtures. Preferred preservatives include parabens, phenoxyethanol or benzalkonium chloride, alone or in combination.

The composition used in the present invention can further contain at last one emollient. Among the emollients whose role is to moisturize the skin and facilitate the application of the formulation, preferably used, but are not limited to, compounds such as glycerin, propylene glycol, dipropylene glycol, propylene glycol dipelargonate, lauroglycol, alone or in combination. A preferred emollient includes propylene glycol.

The composition according to the invention may comprise at least one ingredient selected from the following list:
- at least one gelling agent such as the carbomers preferably Carbopol 974P NF and Carbopol 980 NF,
- at least one chelating agent such as Disodium edentate (EDTA),
- at least one emollient such as propylene glycol,
- at least one wetting agent chosen from poloxamers and preferred poloxamer 124,
- at least one preservative agent such as parabens, phenoxyethanol or benzalkonium chloride alone or in combination.

In a most preferred embodiment of the present invention, the composition is an aqueous gel having the following formulation:
- about 2.5% of BPO;
- about 0.1% of adapalene;
- about 0.10% of disodium EDTA;
- about 4.00% of glycerol;
- about 4.00% of propylene glycol;
- and also, preferably:
- about 0.05% of sodium docusate;
- about 0.20% of poloxamer 124;
- about 4.00% of a gelling agent consisting of acrylamide / sodium acryloyldimethyltaurate copolymer and isohexadecane and polysorbate 80;
- NaOH, in an amount sufficient to obtain a pH of 5;
all amounts being indicated by weight, with regard to the total weight of the composition.

Furthermore, the composition as described above can comprise all the constituents normally present in the type of application envisaged.

The medicament according to the present invention can comprise a large variety of additional components; in particular, they can be absorbents, abrasives, antiacne agents, antifoaming agents, antimicrobial agents, antioxidants, binders, biological additives, buffers, chelating agents, colorants, cosmetic astringents, cosmetic biocides, external analgesics, film-forming agents, fragrance components, opacifying agents, plasticizers, preservatives, other depigmenting agents, emollients, skin-protecting agents, solvents, solubilizing agents, surfactants, agents which absorb ultraviolet light, sunscreens, viscosity-increasing agents (aqueous or nonaqueous), humectants, sequestering agents, and the like.

These additives may be present in the composition in an amount of 0.001% to 20% by weight relative to the total weight of the composition.

### DESCRIPTION OF FIGURES:

Figure 1 represents the mean total lesions count of primary lesions evaluated with VISIA
Figure 2 represents the mean inflammatory and non-inflammatory lesions count evaluated with VISIA
Figure 3 represents the total scarring count evaluated with mECCA grading scale
Figure 4 represents the 3D assessment of scars volume
Figure 5 represents the mean duration of papules related to their evolution - evaluated with VISIA.

The present invention will be now illustrated by the following examples.

### EXAMPLE: Evaluation of the effect of a composition containing a fixed combination of adapalene-BPO compared to its vehicle on the prevention of secondary acne lesions and/or the treatment of pre-existing secondary lesions.

This example provides a demonstration that a composition with a fixed combination of adapalene and BPO provides efficient treatment of atrophic acne scars.

This is an exploratory study to improve the knowledge on the secondary acne lesions by investigating the filiation between primary and secondary ones using clinical and photographic evaluations.

Moreover, as photodamage lesions and atrophic acne scars are linked to the same process of dermal matrix loss, the sponsor anticipates a potential beneficial effect of Adapalene-BPO gel compared to its vehicle to prevent occurrence of secondary acne lesions and to improve the preexisting atrophic acne scars.

As primary objective(s), this study describes and documents the filiation between the primary (Papules, Pustules, Nodules) and secondary acne lesions (Atrophic scars; Post-inflammatory erythema with/ without hyperpigmentation)

As secondary objective(s), this study evaluates the effect of Adapalene-BPO fixed combination compared to its vehicle on the prevention of secondary acne lesions and/or the treatment of pre-existing secondary lesions.

### CLINICAL STUDY PROTOCOL

This was an exploratory, international, multi-centre, randomized, investigator blinded, vehicle controlled study using intra-individual comparison (right half-face versus left half-face), involving approximately 45 subjects with acne vulgaris on face, meeting specific inclusion/non-inclusion criteria.

All subjects attend to:
- a screening period which must be within 4 weeks before Baseline, with a minimum of 2 weeks for women of childbearing potential,
- a 6-month treatment period during which an adapalene-BPO gel is applied on one half-face and its vehicle on the other one, according to a randomization list, once a day in the evening before going to bed, between 5 to 7 days per week;

During the treatment period, subject were attend a visit each day the first week to be trained properly to the study applications (except the weekend), then a biweekly visit during the first two months and two visits per month during the four following months.

The following evaluations were performed:
- A clinical identification of the lesions type that was reported on photographs (VisiaCR from Canfield) so that a filiation between primary and secondary acne lesions was built over time to investigate evolution and resolution of each type of lesions.
- Global scarring grading with the mECCA scale at Baseline, V2 and at the end of each month;
- 3D pictures of regions of interest (ROI) with the 3D Lifeviz™ Microsystem to generate descriptive parameters of scarring, e.g. volume, depth, perimeter at Baseline, and at the end of each month.
- The safety evaluation by the recording of the adverse events (AE) (from Screening visit).

### Filiation of acne lesions

The primary objective of this study is to follow the resolution of the primary lesions of acne to identify the type of lesions involved in the onset of secondary lesions. The physiopathological mechanisms involved in the primary lesions are well-known and largely targeted by the drugs currently on the market. On the contrary, secondary lesions are poorly investigated and not addressed by any treatment. Accordingly, there is a clear medical need for such types of lesions.

In this study, primary lesions will be clinically characterized and followed over time during six months and the resolution of each type of lesions will be documented.

### Product local tolerance

Adapalene-BPO gel (Epiduo® for USA and France or Tactuo® in Canada) is a commercial product that will be used according to the indications and usage of its labelling (see Epiduo® US labelling, Tactuo® Canada Product Monograph and Epiduo® France labelling).

Warnings and precautions with Adapalene-BPO gel are well-known:
Exposure to sunlight, including sunlamps, should be minimized during the use of Adapalene-BPO gel. Patients with high levels of sun exposure and those with inherent sensitivity to sun should exercise particular caution. Use of sunscreen products and protective apparel, (e.g., hat) are recommended when exposure cannot be avoided. Weather extremes, such as wind or cold, may be irritating to patients under treatment with Adapalene-BPO gel.

Local cutaneous reactions including erythema, scaling, dryness, and stinging/burning may be experienced with use of Adapalene-BPO gel. Irritant and allergic contact dermatitis may occur.

The product should not be applied to cuts, abrasions, eczematous or sunburned skin. As with other retinoids, use of "waxing" as a depilatory method should be avoided on skin treated with Adapalene-BPO gel. Concomitant use of other potentially irritating topical products (medicatedor abrasive soaps and cleansers, soaps and cosmetics that have strong skin-drying effect and products with high concentrations of alcohol, astringents, spices, or limes) should be avoided.

### Study duration

Sponsor considers that this treatment duration is supported by significant data of long-term use of Adapalene-BPO (Pariser, 2007).

Since acne is a chronic and long-lasting skin condition, the Sponsor considers that 6 months of treatment will be sufficient to obtain clinical effect on the basis of the following points:
- Scarring and post-inflammatory hyperpigmentation (PIH) appear secondarily to the primary lesions of acne defined by non-inflammatory and/or inflammatory lesions and may correspond to a resolution of these primary lesions;
- It is generally agreed in acne clinical trials that 3 months of treatment is sufficient to evaluate the efficacy on the primary lesions of acne. Consequently, to investigate their resolution, the duration of treatment has to be increased;
- Finally, taken into account that remodeling processes are generally described to occur slowly, this period of time is considered long enough to induce a significant stimulation of the dermal fibroblasts and collagen production, that could be clinically observable or physically measurable.

### Comparators choice

This is an exploratory study designed to investigate the filiation between acne lesions and to evaluate the potential effect of an adapalene-BPO gel on pre-existing or new scars. No products
have been described to have an efficacy on scars. Then, to fulfil the study objectives, the best way remains to compare the treatment to its vehicle.

### Intra-individual design and sample size

As the main objective of this clinical trial is to describe and document the natural history of secondary lesions, the population size has been calculated according to the second objective of the study. It was based on past split face trials with Adapalene-BPO versus its vehicle (right versus left). This design is appropriate to increase the study power and to minimize the total number of subjects by using the intra-individual variability instead of inter-individual variability for comparing the evolution of primary and secondary lesions and the effect of the treatment versus its vehicle.

**Table 1: Study flow chart**

| **PROCEDURES** | | **Treatment period** - **Study visits** | | | | | | | | | | | |
|---|---|---|---|---|---|---|---|---|---|---|---|---|---|
| | **Screening period** | **Week 1 V1** | **Week 1 V2** | **Week 2 V3/V4** | **Week 3 V5/N6** | **Week 4 V7/N8** | | **Week 8 V15 / V16** | | **Week 12 V18** | **Week 16 V20** | **Week 20 V22** | **Week 24 V24** |
| Demographics/Medical history | x | X | | | | | | | | | | | |
| Previous treatment/Procedure | x | | | | | | | | | | | | |
| Concomitant treatment/Procedure | x | X | X | X | X | X | | X | | X | X | X | X |
| Product application | | Once a day on site except WE | | Once a day, between 5 to 7 days per week at home | | | | | | | | | |
| Lesions filiations on photography (Visia CR) | | X | X | X | X | X | X | | X | | X | X | X |
| Scars assessment (mECCA scale) | | X | X | | | X | X | | X | | X | X | X |
| 3D photographiy (lifeviz) | | X | | | | X | X | | X | | X | X | X |
| Adverse events ^{d} | X | X | | X | | X | X | | X | | X | X | X |

| | | | | | | | | | | | | | |
|---|---|---|---|---|---|---|---|---|---|---|---|---|---|
| d. Adverse event onsets after subject signature of the informed consent form should be recorded on the AE form of the CRF. | | | | | | | | | | | | | |

### SELECTION AND DISPOSITION OF CLINICAL TRIAL POPULATION

### 1 Number of subjects

As a screen failure rate of approximately 20 percent is expected, approximately 54 subjects may have to be screened in order to get 45 subjects randomized.

### 2 Clinical trial population characteristics

In order to be eligible for the clinical trial, subjects must fulfil all of the following criteria. Some criteria are to be checked at Screening visit and/or at Baseline visit, as specified.

### 2.1 Inclusion criteria

1. The subject is a male or female aged from 18 to 35 years inclusive (Screening).
2. The subject has a clinical diagnosis of an active moderate *acne vulgaris* on face defined by (Baseline):
   - A minimum of 20 but not more than 40 inflammatory lesions (papules and pustules) (excluding the nose)
   - No more than one acne nodule;
   - A minimum of 10 atrophic acne scars (upper than 1.5mm) (excluding the nose).
3. The subject has a symmetric number of inflammatory lesions, closed comedones and scarrings on the whole face i.e. there are no more than twice as many lesions on one half of the face than on the other half (Baseline).
4. The subject has a skin phototype of I to IV on Fitzpatrick's scale (Fitzpatrick T.B., 1993) (skin phototypes V and VI are excluded from this study because of the difficulty of counting lesions on dark skin) (Do, 2008) (Screening).
5. If the subject is a woman of childbearing potential, she agrees to use an effective method of contraception for at least one month prior to the Screening visit, during the study and until one month after the end of the study, with one of the following methods (Screening and Baseline):
6. If the subject is a woman of non-childbearing potential, she has to be postmenopausal (absence of menstrual bleeding for at least one year prior to the enrolment), or permanently sterilized (had hysterectomy or bilateral oophorectomy) (Screening).
7. The subject is willing and able to comply with the requirements of the protocol. In particular, subject must adhere to the visit schedule, concomitant therapy prohibitions, and must be compliant to the treatment (Screening and Baseline).
8. The subject has understood and signed an Informed Consent Form (ICF) approved by the Independent Ethics Committee (IEC) or Institutional Review Board (IRB), prior to any investigational procedure (Screening).

### 2.2 Non-inclusion criteria

1. The subject has a secondary acne form (chloracne, drug-induced acne, etc.) (Screening).
2. The subject has a severity of acne that is not amenable to treatment with Adapalene-BPO (Screening).
3. The subject has more than 3 excoriated acne lesions at screening visit.
4. The subject has an underlying disease, surgical or medical condition, which in the opinion of the investigator, could put the subject at risk as uncontrolled chronic or serious diseases which would normally prevent participation in any clinical trial (e.g. cancer, AIDS, diabetes, severe hepatic or renal impairment, severe cardiopathy, or obesity defined by a Body Mass Index (BMI) upper to 30) (Screening).
5. The subject is a man with beard or facial hair, which would interfere with clinical evaluation or clinical procedure (Screening).
6. The subject has eczema or seborrheic dermatitis on the face or other underlying dermatological disease, which, in the judgement of the investigator, could interfere with the subjects safety or the study assessments (Screening).
7. The subject has a known or suspected allergy to one of the test products (see Epiduo® labeling) (Screening).
8. The subject presents with positive serology results for HIV, or HBsAg or HCV at Baseline visit.
9. The subject is a woman who has a positive urine pregnancy test (UPT having a sensitivity down to at least 25 IU/ml for hCG) or is breast-feeding or is planning a pregnancy during the course of study and up to one month following the end of study (Screening, Baseline).
10. The subject has used prohibited topical or systemic treatments without sufficient protocol defined wash-out prior to Day 1/Baseline or is unwilling to refrain from use during the study (Screening and Baseline) such as Topical treatments on face like Antibiotics, benzoyl peroxide, azelaic acid, corticosteroids, hydroxyacids, Zinc containing treatments, antibacterials, antiseptics, other anti-inflammatory treatments or other acne treatments, Retinoids, Microdermabrasion or laser resurfacing on the face or Systemic Treatments like Corticosteroids (inhaled administration is not considered as a systemic route), Antibiotics, Retinoids, isotretinoin.

### CLINICAL SUPPLIES

### 1. Method of treatment assignment

Prior to the start of the study, a randomization list will be generated for each centre, by a statistician from Galderma R&D or designee and will be transmitted to the assigned clinical packaging organization.

The RANUNI routine of the Statistical Analysis System (SAS) system will be used to randomly assign the active or the vehicle on the right or left side of the face.

The 'left' or 'right' side of the face is always defined as the subject's left or right.

The active treatment and the vehicle will be coded on the list (e.g A and B) and the randomization list will be provided to the site with the clinical supplies.

At Baseline visit (V1), each subject who fulfils all inclusion/non-inclusion criteria will be assigned a Randomization Number (3-digit number). This randomization number will be allocated in ascending sequential order to each subject and no number should be omitted or skipped.

### 2 Instructions for use and Administration

The study treatment dispenser gives each subject verbal and written instructions on how to use the study treatment at on-site visit. Subject kit is dispensed according to the chronological order of enrollment of subjects into the study.

The designated study personnel in charge of the study medications (designated as the study drug dispenser) will be different from the Study Evaluator in order to keep the blind.

She/he will give each Subject verbal and written instructions on how to use the investigational and non-investigational products. In addition, she/he will show to the Subject how to perform the Study gel application at Baseline visit, during the first week on site and every time it will be necessary, when the applications will be performed by the subject itself at home.

Subjects will treat one half-face with Adapalene-BPO gel and the other one with its vehicle, once.

Products will be applied as described above:
- Apply a thin film of products to each half-face, once daily after washing, by using a pea-sized amount for the upper part of the half-face (defined by the half-forehead and the temple) and a second pea-sized amount for the lower part of the half-face (defined by the cheek and the mandibular area).
- The Subject will wear single-use gloves, and these must be changed before conducting the same mode of application on the other half-face with the second study product.
- Exclude the median line of the face defined from the forehead to the chin through the nose and dividing the face in two half-faces. Avoid the eyes, lips and mucous membranes.

The treatment administration is further described below:

| | **Investigational Product** | **Comparator** |
|---|---|---|
| Concentration | 0.1%/2.5% | NA |
| Dose Regimen | One daily application after washing in the evening, 5 to 7 days per week | |
| Period of Administration | 6 months | |
| Route of Administration | Topical | |
| Location of treated areas | Half-face | |

### 3 Treatment compliance management and record

### 3.1 Generalities

At baseline, Visit 8 (Week 4), Visit 16 (Week 8), Visit 18 (Week 12), Visit 20 (Week 16) and 22 (Week 20), a treatment calendar was given to each subject in order to record the study applications compliance.

The study treatment dispenser reviewed with the Subject the treatment calendar at every post- Baseline on-site visit and questioned the Subject regarding the application technique, frequency and missed doses of study drugs and the use of any other additional topical, systemic medical treatment as well as OTC treatments.

If necessary, the study treatment dispenser reinstructs the subject regarding the application of the study drugs and the compliance.

At the end of the study, Study drugs were returned to the Sponsor to be weighted to quantify the extent of exposure.

### 3.2 The Clinical Compliance Control System (CCCS)

Due to the particularity of the split face study design and the duration of the treatment period, inconsistent with the management of the application on site by a qualified person, the sponsor proposes to manage the risk of side error, associated to the split face application with the use of the Clinical Compliance Control System (CCCS from Triacys).

CCCS is a device allowing to control and to record the applications of study drugs by patients at home.

CCCS comes in the form of a battery-operated briefcase and includes a mirror in which is embedded a video camera.

The images during drug applications are recorded through a tactile screen, the patient is guided when applying drugs to avoid side errors.

CCCS is equipped with:
- A bar code reader to identify precisely the treatments.
- A temperature and humidity sensor.
- An internal clock to record date and time of each drug application.
- A USB port to send data through an Internet connection.
- A space to house therapeutic units.

### Course of a CCCS session:

Patient is asked to undertake the application of the gel in front of the video camera. All data (video, date and time, temperature, humidity, etc...) are recorded. The briefcase is closed at the end of the operation.

A central server is in place to retrieve all data at each session or visit at the investigator site. A compliance report is established after validation by independent operators. For each patient, a compliance index is calculated to be used to perform correlations with clinical efficacy and safety of use of the drug.

No image will be archived. At the end of the study, all of them will be destroyed.

### CLINICAL TRIAL ASSESSMENT

Throughout the study, when possible, the evaluations for each individual subject are performed by the same evaluator. In the event there is a change in the assigned evaluator for a given subject, the reason for change must be documented. If it is not possible to use the same evaluator to follow a given subject, the Sponsor recommends that evaluations between the primary and subsequent evaluator overlap (both evaluators should examine the subject together and discuss findings) for at least one visit.

### 1 Filiation between primary and secondary acne lesions by photography

### 1.1 Visia CR system

Standardized digital photographs of the whole face (front, right and left) were taken during each study visit using a Canfield Scientific stereostatic device (Visia CR®).

Photographs were taken at Baseline, twice a week during the first two months with two or three days between each visit and once every two weeks during the last four months. In practice, the subjects can be randomized every day except Wednesday.

The visits rate during the first two months is guided by the mean duration of the two types of primary lesions assessed by the sponsor to be of about 5 days for the inflammatory lesions and about 13 days for the non-inflammatory lesions.

Hence, all the lesions occurring during this period were identified and tracked by the evaluator (Investigator or designee).

The investigator/evaluator identifies the different primary and secondary acne lesions separately on each half face using both visual observation and palpation.

Primary lesions are defined by:
- Inflammatory lesions
   - Papule - A small, red, solid elevation less than 1.0 cm in diameter.
   - Pustule - A small, circumscribed elevation of the skin that contains yellow-white exudate.
   - Nodule - A circumscribed, elevated, solid lesion generally more than 1.0 cm in diameter with palpable depth.
- Non-inflammatory lesions
   - Closed Comedone - A tiny white papule (whitehead).

Secondary lesions are defined by:
- Atrophic scars
- Post-inflammatory erythema without hyperpigmentation
- Post-inflammatory erythema with hyperpigmentation
- Others (precise in the comment section of the CRF)

### 1.2 Filiation process

Two solutions are proposed to assess this filiation (at Site discretion):
- By the reporting of each type of lesions on the printed photographs shot at each visit using the following color code:
   - Red: Pustules
   - Orange: Papules
   - Blue: Closed comedones
   - Black: Atrophic scars
   - Light blue: Post-inflammatory erythema without hyperpigmentation
   - Green: Post-inflammatory erythema with hyperpigmentation
   - Yellow: Others (precise in the comment section of the CRF)

By the end of the study, all the information collected on the printed photographs will be captured in Skinpad software after a step of realignment of the images with a dedicated software (see below).

Directly on computer by using dedicated softwares developed by Galderma R&D: alignment software and Skinpad software.

### 1.2.1 Alignment Software

During the study, a lot of photographs were be taken. But from one visit to another, and even in the same visit from one modality to another, the subject did not have exactly the same position in front of the camera.

A dedicated alignment software was developed by Galderma in order to be able to provide a set of images that are all aligned and easily comparable. It will transform the images and calculate a new image that is "aligned" with a reference image:
When all the images are aligned, the software produces a set of aligned images for Skinpad

### 1.2.2 Skinpad software

This software is able to:
- View dynamically a set of images (for one patient, several zone, several modality)
- Define an area for counting
- To mark a lesion and to qualify the lesion at each visit.

Whatever the chosen process by the site, a dedicated person (Investigator or designee) is in charge of counting all the lesions (primary and secondary) identified by the evaluator after each visit and report the information in the appropriate CRF pages.

### 2. Clinical assessment

At baseline, at visit V2 and once a month, the evaluator (Investigator or designee) performs a global or a semi-quantitative evaluation of the severity of scarring using a new scale for atrophic scars evaluation.

The mECCA grading scale (m for modified) is derived from the original ECCA (for Echelle devaluation Clinique des Cicatrices d'Acne) designated to assess easily and quickly the severity of acne scars in current practice (Dreno B, et al. 2007. ECCA grading scale: an original validated acne scar grading scale for clinical practice in dermatology. Dermatology. 214:46-51).

The scale presents:
▪ A Global Assessment of scars defined by:

| **Category** | Score | Description |
|---|---|---|
| **Clear** | **0** | No visible scars from acne |
| **Almost Clear** | **1** | Hardly visible scars from 50 cm away |
| **Mild** | **2** | Easily recognizable; less than half the affected face area involved |
| **Moderate** | **3** | More than half and less than 75 % of the affected face area involved |
| **Severe** | **4** | More than 75 % of the affected face area involved |

▪ A semi-quantitative evaluation by facial area:
Facial area definition: 6 facial areas are the forehead, the right and left temples, the right and left cheeks and the mandibular area (temples are the facial areas defined by the inferior part of orbicular areas to implantation of hairs, top superior part of orbicular areas to implantation of hairs (based of bone); Mandibular: facial area as from corner of mouth to bottom of ear opening.

The surface affected by the presence of scars on each area is quantified according to the following categories:
- 0=0%
- 1 = 1 to 25% (<¼ area)
- 2 = 26% to 50% (up to ½ area)
- 3 = 51% to 75% (up to ¾ area)
- 4 = 76% to 100% (entire area)

The atrophic scars are counted according to their size defined in 2 categories (Jacob et al, 2001):
- Atrophic scars 2-4 mm: includes boxcar (sheer edges), rolling (irregular surface), and undetermined types
- Atrophic scars > 4 mm: includes boxcar (sheer edges), rolling (irregular surface), and undetermined types.
- Atrophic scars < 2 mm (icepick punctiform) will not be counted as it has been shown in a preliminary work that there is a large variability of counting between scars evaluators (internal data).

Scars must be counted by the same evaluator for the same patient. The evaluated areas must be examined under the same conditions of light exposure at each visit (same angle to avoid shadow and same light source, i.e. artificial or daylight).

### 3. 3-D photographic assessment

3-D pictures are performed with the digital 3D Lifeviz™ Microsystems at Baseline and once a month at the investigational site by the same dedicated person throughout the study.

At Baseline and for each subject, 2 or 3 ROI by hemi-face (3 by 3cm area) is defined. It should contain at least 5 atrophic acne scars whatever the type [ice pick (0.5 < 1.5mm), boxcar (1.5-4mm) or rolling (>4mm)]. A transparent mask of repositioning is used to localize this ROI and is placed in the corresponding CRF. This template is used each time this assessment is performed.

All 3-D images are analyzed to provide different descriptive parameters of scarrings (e.g volume, depth or perimeter) used to evaluate the effects of treatment over time.

### 4. Safety assessment

A safety assessment was conducted for all subjects at the screening visit (from the Informed consent signature) and every subsequent visit. The safety parameters are AEs, to be recorded.

Laboratory safety tests and pregnancy tests are drawn at screening visits. Pregnancy tests are conducted also at baseline and once a month during the treatment period.

Adverse events (AEs) are monitored throughout the course of the clinical trial. All AEs are to be reported on the Adverse Event Form of the CRF with complete information as required. If AEs occur, the main concern will be the safety of the subjects. At the time of the ICF signature, each subject must be provided with the name and phone number of clinical trial center personnel for reporting AEs and medical emergencies. Adverse events definition is according to ICH E2A document. An AE is any untoward medical occurrence in a subject or clinical investigation subject administered a pharmaceutical product and which does not necessarily have a causal relationship with this treatment. An AE can therefore be any unfavorable and unintended sign (including an abnormal laboratory value), symptom, or disease temporally associated with the use of a medicinal (investigational) product, whether or not related to the medicinal (investigational) product.

Thus any new sign, symptom or disease, or any clinically significant worsening of an existing sign, symptom or disease compared to the condition at the first visit (including disease treated), should be considered as an AE. Lack of efficacy is not considered as an AE.

### CLINICAL TRIAL VISITS DESCRIPTIONS AND PROCEDURES

### DESCRIPTION OF CLINICAL TRIAL VISITS

Refer to the Schedule of Assessments table in the Synopsis **(Erreur! Source du renvoi introuvable.).**

A written, signed ICF including HIPAA/PIPEDA and Photo Consent Form was obtained prior to performing any clinical trial-related evaluations and/or procedures.

During the first two months, there is a visit every two or three days.

### SCREENING VISIT

Screening visit takes place within maximum 4 weeks prior the baseline visit (V1). During this visit, the Investigator or a qualified staff member:
▪ explains the nature of the study in detail to the subject, particularly the prohibited concomitant therapies and the constraints of the study (such as restrictions in the use of topical or systemic prescription, uv exposure, etc.).
▪ has the subject read, dated and signed the informed consent form. give a copy to the subject and file the other copy in the investigator site file;
▪ assigns the subject a subject's identification number (crf number).
▪ records demographics, relevant medical history,
▪ verifies inclusion and non-inclusion criteria ;
▪ questions the subject about previous and concomitant therapies and procedures;
▪ performs a upt for women of childbearing potential;
▪ performs a blood sampling for laboratory tests (virology)
▪ completes the subject screening & enrolment log.
▪ gives the subject an appointment for the beginning of the treatment period (v1).

### ON-TREATMENTS VISIT (FROM V1 TO V24)

At every post-screening on-site visit, the investigator:
▪ asks the subject by an open-ended question, records all events, as appropriate,
▪ asks the subject about any changes in his/her concomitant therapies/procedures (added, removed or changed) since the previous visit. documents all changes on the concomitant and procedures form of the crf accordingly.

### BASELINE VISIT (V1) AND VISIT v2

during the baseline visit, the investigator or her/his designee:
▪ re-checks inclusion/non-inclusion criteria (e.g.: respect of wash-out periods and serology results among others);
▪ performs a UPT for women of childbearing potential;
▪ Confirms the subject's randomization;
▪ Takes photographs of the entire face using the Visia-CR system
▪ Performs the identification and the characterization of primary and secondary acne lesions;
▪ Performs the scars assessment using the mECCA grading scale;
▪ Identifies 2 or 3 ROI by half-face for each subject and take 3D photographs using the digital 3D Lifeviz™ Microsystems.

The study treatment dispenser:
▪ Presents and explains to the subject the application procedure of study products.
During the first week, the subject comes back to the site each day except the weekend to be trained properly to the procedures of study applications and the use of the CCCS.

During the visit V2, the Investigator or her/his designee:
▪ Takes photographs of the entire face using the Visia-CR system
▪ Perform sthe identification and the characterization of primary and secondary acne lesions;
▪ Performs the scars assessment using the mECCA grading scale

### FROM VISIT V3 TO VISIT V23

At each visit, the investigator or her/his designee:
▪ Takes photographs of the entire face using the Visia-CR system
▪ Performs the identification and the characterization of primary and secondary acne lesions;

In addition to the procedures listed above, once a month on V8 (week 4), V16 (week 8), V18 (week 12), V20 (week 16), and V22 (week 20), the investigator or her/his designee performs:
▪ the scars assessment using the mECCA grading scale;
▪ 3D photographs of the ROI using the digital 3D Lifeviz™ Microsystems ;
▪ a UPT for women of childbearing potential;

At the same visits, the study treatment dispenser insures the management of the drugs dispensing and the control of the application procedure.

### FINAL/EARLY TERMINATION VISIT (V24)

At this visit or in case of early termination, the investigator or her /his designee carries out the following actions:
▪ Ask the subject about AEs by an open-ended question, such as "Have you noticed any change in your health since the last visit?" Record all events, as appropriate, on the corresponding CRF pages;
▪ Ask the subject about any changes in his/her concomitant therapies/procedures (added, removed or changed) since the previous visit. Document all changes on the concomitant and procedures form of the CRF accordingly;
▪ Perform a UPT for women of childbearing potential.
▪ Take photographs of the entire face using the Visia-CR system
▪ Perform the identification and the characterization of primary and secondary acne lesions;
▪ Perform the scars assessment using the mECCA grading scale ;
▪ Take 3D photographs of the ROI using the digital 3D Lifeviz™ Microsystems .
▪ Control the returning of the last study drugs

During this visit, the Exit form should be completed and signed by the Principal investigator.

### STATISTICAL METHODS

A Statistical Analysis Plan (SAP) contains a detailed and technical description of specific data conventions, calculations and of statistical procedures for executing the analyses that are specified in the sections of the clinical trial protocol.

### Populations analyzed, evaluability and limitation / evaluation of bias:

### 1. Intent-to-treat (ITT) Efficacy Population

The ITT Population is defined as comprising all subjects who are randomized. All primary efficacy variables and secondary efficacy variables will be analyzed based on the ITT Population.

### 2.Per-protocol (PP) Efficacy Population

The PP Population is defined as comprising the ITT subjects who have no major protocol deviations.

### 3. Safety Population

The Safety Population is defined as comprising the ITT Population subjects who applied the study drug(s) at least once. In practice, only the subjects who return their study drug(s) unopened will be excluded from the Safety Population. All safety data will be summarized based on the Safety Population.

### Inferential statistical analyses

Subject disposition, demographics, baseline characteristics, previous therapies, concomitant therapies, and treatment duration by treatment are summarized by descriptive statistics.

A transition matrix by lesion status between successive observation periods is made; pre-existing lesions, new lesions (atrophic scars, post-inflammatory erythema without hyperpigmentation and post-inflammatory erythema with hyperpigmentation), clearance of lesions and their type are counted. This count was also performed on each treated half face, by treatment.

### CONCLUSIONS OF THE STUDY

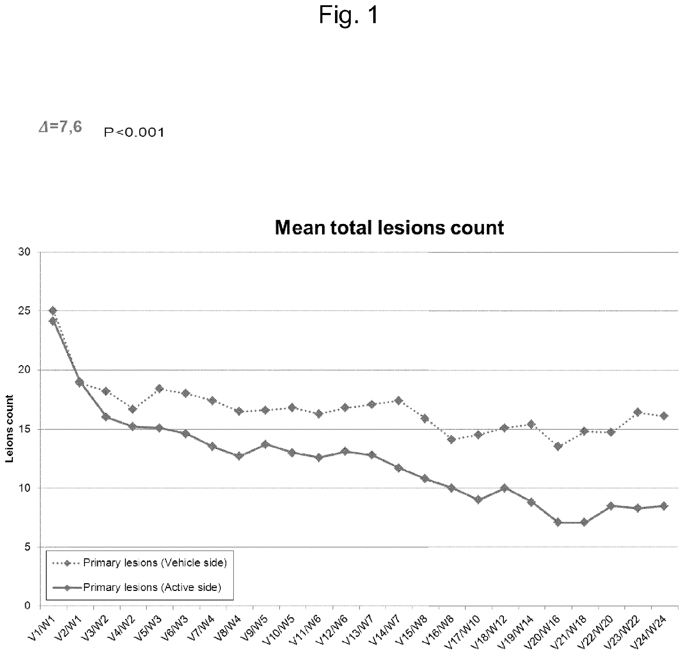

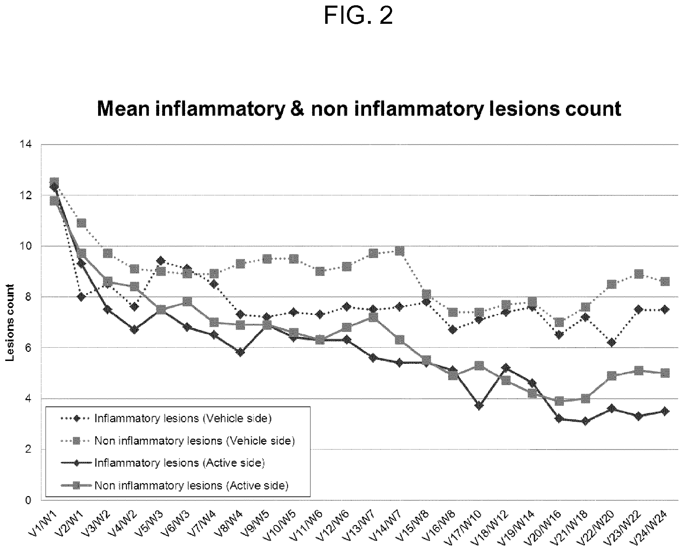

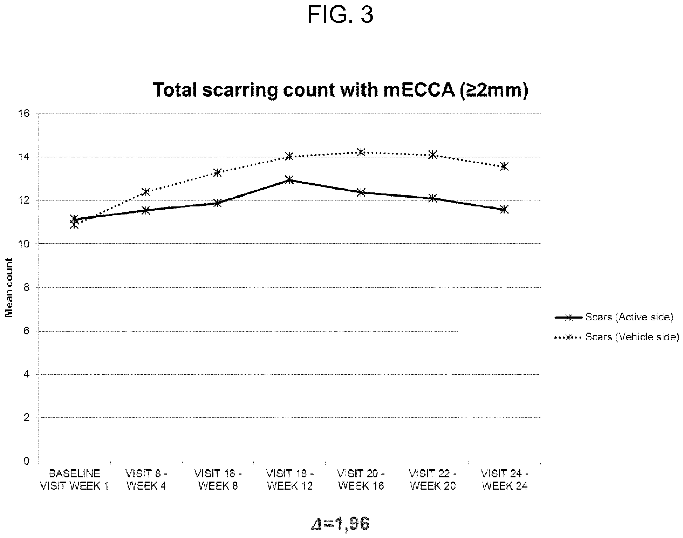

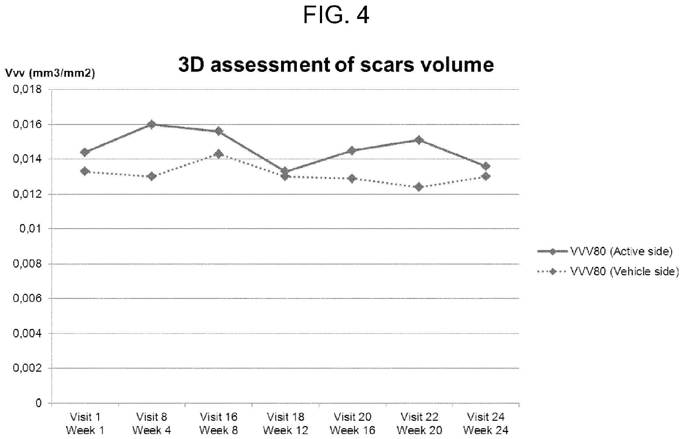

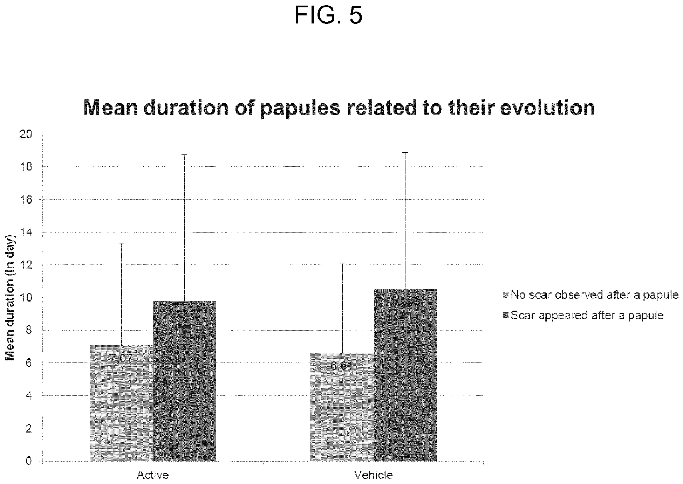

The results of the study are illustrated in Figures 1 to 5.

This study demonstrates the effect of an adapalene-BPO fixed dose combination on atrophic acne scars.

This adapalene-BPO fixed dose combination is superior to its vehicule on atrophic scars. 3D results did not show any effect on pre-existing scars but suggest a preventive effect of this fixed combination. Over 24 week period treatment Adapalene-BPO fixed combination stabilized scar counts whereas scars continued to increase with vehicule (the difference Δ is about 2 scars.

More precisely, the results show that Epiduo is efficient on acne scars with demonstration of:
- Effect size at week 24 : Δ = -1.96 for scar, Δ= -4 for inflammatory lesions, Δ = -3 for Non inflammatory lesions
- Visible on SGA : switch from mild to almost clear (p=0.0032)

Papule duration is statistically longer when the papule evolves in scar suggesting there is a different acne lesion cycle behind scar physiopathology with a more severe inflammation.

## Claims

1. Composition containing a fixed combination of adapalene or a salt thereof and benzoyl peroxide for use in preventing acne atrophic scars over a period treatment of 24 weeks.

2. The composition for use according to claim 1, wherein said composition is applied onto the scarred skin.

3. The composition for use according to claims 1 or 2, wherein said composition comprises from 0.01% to 2%, preferably from 0.05% to 0.5% and more preferably from 0.1% to 0.3% of adapalene, by weight with regard to the total weight of the composition.

4. The composition for use according to claims 1 to 3, wherein said composition comprises from 0.1% to 5%, preferably from 0.5% to 5%, more preferably from 2% to 5%, and most preferably 2.5% of benzoyl peroxide, by weight with regard to the total weight of the composition.

5. The composition for use according to claims 1 to 4, wherein said composition is in the form of a gel, an emulsion, or a lotion, and preferably in the form of an aqueous gel.

6. The composition for use according to claim 5, which is in the form of an aqueous gel having the following composition, expressed in percentages by weight with respect to the total weight of the composition:
- adapalene: from 0,1% to 0,3%,
- benzoyl peroxide: from 2.5% to 5%,
- at least one gelling agent: from 0.001% to 15% and more preferentially from 0.15% to 5%,
- at least one chelating agent: from 0,01% to 1,5%,
- at least one preservative agent: from 0,01% to 3%,
- at least one wetting agent: from 0.1% to 10% , and
- at least one pH adjuster.

## Patentansprüche

1. Zusammensetzung, enthaltend eine fixe Kombination von Adapalen oder einem Salz davon und Benzoylperoxid zur Verwendung bei der Prävention von atrophen Aknenarben über eine Behandlungsperiode von 24 Wochen.

2. Zusammensetzung zur Verwendung nach Anspruch 1, wobei die Zusammensetzung auf die vernarbte Haut aufgetragen wird.

3. Zusammensetzung zur Verwendung nach Anspruch 1 oder 2, wobei die Zusammensetzung 0,01 Gew.-% bis 2 Gew.-%, vorzugsweise 0,05 Gew.-% bis 0,5 Gew.-% und besonders bevorzugt 0,1 Gew.-% bis 0,3 Gew.-% Adapalen umfasst, bezogen auf das Gesamtgewicht der Zusammensetzung.

4. Zusammensetzung zur Verwendung nach den Ansprüchen 1 bis 3, wobei die Zusammensetzung 0,1 Gew.-% bis 5 Gew.-%, vorzugsweise 0,5 Gew.-% bis 5 Gew.-%, besonders bevorzugt 2 Gew.-% bis 5 Gew.-% und am meisten bevorzugt 2,5 Gew.-% Benzoylperoxid umfasst, bezogen auf das Gesamtgewicht der Zusammensetzung.

5. Zusammensetzung zur Verwendung nach den Ansprüchen 1 bis 4, wobei die Zusammensetzung in Form eines Gels, einer Emulsion oder einer Lotion und vorzugsweise in Form eines wässrigen Gels vorliegt.

6. Zusammensetzung zur Verwendung nach Anspruch 5, welche in Form eines wässrigen Gels mit der folgenden Zusammensetzung, ausgedrückt in Gewichtsprozent bezogen auf das Gesamtgewicht der Zusammensetzung, vorliegt:
- Adapalen: 0,1% bis 0,3%,
- Benzoylperoxid: 2,5% bis 5%,
- mindestens ein Geliermittel: 0,001% bis 15% und besonders bevorzugt 0,15% bis 5%,
- mindestens einen Chelator: 0,01% bis 1,5%,
- mindestens einen Konservierungsstoff: 0,01% bis 3%,
- mindestens ein Netzmittel: 0,1% bis 10% und
- mindestens ein Mittel zum Einstellen des pH-Wertes.

## Revendications

1. Composition contenant une combinaison fixée d'adapalène ou d'un sel de celui-ci et de peroxyde de benzoyle pour son utilisation dans la prévention de cicatrices atrophiques d'acné en un traitement d'une durée de 24 semaines.

2. Composition pour utilisation conforme à la revendication 1, dans laquelle ladite composition est appliquée sur la peau marquée de cicatrices.

3. Composition pour utilisation conforme aux revendications 1 ou 2, laquelle composition comprend de 0,01 à 2 %, de préférence de 0,05 à 0,5 %, et mieux encore de 0,1 à 0,3 %, d'adapalène, en poids rapporté au poids total de la composition.

4. Composition pour utilisation conforme aux revendications 1 à 3, laquelle composition comprend de 0,1 à 5 %, de préférence de 0,5 à 5 %, plus préférentiellement de 2 à 5 %, et encore plus préférentiellement 2,5 % de peroxyde de benzoyle, en poids rapporté au poids total de la composition.

5. Composition pour utilisation conforme aux revendications 1 à 4, laquelle composition se présente sous la forme d'un gel, d'une émulsion ou d'une lotion, et de préférence, sous la forme d'un gel aqueux.

6. Composition pour utilisation conforme à la revendication 5, qui se présente sous la forme d'un gel aqueux dont la composition, exprimée en pourcentages en poids rapportés au poids total de la composition, est la suivante :
- adapalène : de 0,1 à 0,3 %,
- peroxyde de benzoyle : de 2,5 à 5 %,
- au moins un agent gélifiant : de 0,001 à 15 %, et de préférence, de 0,15 à 5 %,
- au moins un agent chélatant : de 0,01 à 1,5 %,
- au moins un agent conservateur : de 0,01 à 3 %,
- au moins un agent mouillant : de 0,1 à 10 %,
- et au moins un agent d'ajustement du pH.
